# EUROPEAN PATENT SPECIFICATION

(11) **EP 3 071 197 B1**
(45) Date of publication and mention of the grant of the patent: **25.10.2017**
(21) Application number: 13814647.7
(22) Date of filing: 26.09.2013
(51) Int. Cl.: A61K 31/185, A61K 31/4045, A61K 33/30, A61P 27/16, A23L 33/10, A23L 33/16

(54) **FOOD FOR SPECIAL MEDICAL PURPOSES OR FOOD SUPPLEMENT FORMULATION FOR TREATING HEARING DISTURBANCES**
NAHRUNGSMITTEL FÜR SPEZIELLE MEDIZINISCHE ZWECKE ODER NAHRUNGSERGÄNZUNGSMITTELFORMULIERUNG ZUR BEHANDLUNG VON STÖRUNGEN DES GEHÖRS
ALIMENT UTILISABLE À DES FINS MÉDICALES SPÉCIALES OU FORMULATION DE COMPLÉMENT ALIMENTAIRE DESTINÉE À TRAITER DES TROUBLES DE L'OUÏE

(43) Date of publication of application: 28.09.2016
(73) Proprietor: OCEAN FARMA S.r.l, 04100 Latina (IT)
(72) Inventor: ANNALORO, Raffaele, I-00040 Pomezia (IT); SONAGLIA, Achille, I-00040 Pomezia (IT)
(74) Representative: Cioncoloni, Giuliana
(86) International application number: PCT/IT2013/000258
(87) International publication number: WO 2015/044968

(56) References cited:
- US-A1- 2002 061 870

## Description

The present invention relates to a food for special medical purposes or food supplement for the treatment of hearing disturbances, particularly tinnitus.

Tinnitus is an irritating and often distressing condition of the ear that is characterized by a jingle, real whistle or ringing sound and is one of the most disabling symptoms leading to a pathological condition of the ear. It can manifest as a rustle, clink or hiss, localized to one ear or both ears, which is exacerbated in a quiet environment, so as to interfere with sleep, mood and daily activities. Patient's ability to tolerate tinnitus varies widely. It is one of the most common hearing disorders, affecting 17% of the general population and 33% of the elderly. However, its aetiology and pathophysiology has not yet been clarified.

A number of medications and nutritional supplements are being proposed and sold for the treatment of hearing disturbances, particularly tinnitus, but none of them have been proven to be really efficacious. US 2002/061870 discloses a list of substances sometimes used as dietary supplement that influence the hearing apparatus. A preliminary study has found that vitamin B12 deficiency is common among people with tinnitus, so that vitamin B12 injections have been recommended for such a disorder. *Ginkgo Biloba* extracts have also been used to treat tinnitus, but with poor results.

Zinc deficiency has also been thought to be linked to the development of tinnitus. Zinc is a trace mineral that has many functions in the body. Zinc deficiency is known to cause a variety of neurological disorders, as well as immune dysfunction and skin disorders. Significant correlation between tinnitus and decreased zinc levels as well as reduction in severity of tinnitus after zinc therapy has been reported in some clinical studies, but the effectiveness of zinc treatments were not demonstrated. The body cannot produce or store zinc, so this one must be consumed regularly. Zinc plays an important role in several biochemical and physiologic processes, such as protein synthesis, and in preventing medical conditions, including the common cold and age-related macular degeneration. Zinc is contained in a variety of foods, including oysters, chicken, kidney, beans. It is also freely available as a dietary supplement formulated in capsules and tablets. Zinc is not, however, a standard treatment for tinnitus, so medical advice for its use for this condition must be sought.

Lipoic acid (LA hereinbelow), also known as alpha-lipoic acid (ALA hereinbelow), is an organosulphur compound derived from octanoic acid. The molecule of ALA exists as two enantiomers: (R)-(+)-lipoic acid (RLA hereinbelow) and (S)-(-)lipoic acid (SLA hereinbelow) and as a racemic mixture (R/S)-lipoic acid (R/S-LA hereinbelow). (R)-(+)-enantiomer exists in nature and is an essential cofactor of four mitochondrial enzyme complexes. Both RLA and R/S-LA are available as nutritional supplements and have been used nutritionally and clinically since the 1950s for various diseases and conditions. ALA is present in almost all foods, but slightly more in liver, kidney, heart, spinach, broccoli and yeast extract. Naturally occurring lipoic acid is always covalently bound and not readily available from dietary sources. Additionally, the amount of lipoic acid present in dietary sources is very low. As a result, all lipoic acid available as a supplement is chemically synthesized. Today, R/S-LA and RLA are widely available as nutritional supplements in the form of capsules, tablets and aqueous liquids, and have been branded as antioxidants. ALA converts glucose (blood sugar) into energy. ALA is thought to work as an antioxidant (a substance that neutralizes potentially harmful chemicals called free radicals) in both water and fatty tissue, enabling it to enter all parts of a nerve cell and protect this one from damage. Free radical scavengers are compounds capable of inactivating free radicals. A nutritional supplements manufacturer states that ALA protects against free-radical damage, supports nervous system functions and plays an essential role in generating mitochondria in the hair cells of the inner ear. Another source states that ALA supports nervous system functions and plays an essential role in generating mitochondria in the hair cells of the inner ear. It has also been shown to improve overall energy and age-related hearing losses. As it has such a powerful action against free-radicals, there is also evidence that ALA plays an important role in reducing presbycusis and improving cochlear function, as well as providing protection against noise and auditory toxicity caused by ototoxic drug therapies.

Melatonin is a hormone secreted by the pineal gland in the brain. Melatonin helps regulate other hormones and maintains the body's circadian rhythm. Circadian rhythm is an internal 24-hour clock that plays a critical role in when one falls asleep and when one wakes up. When it is dark, one's body produces more melatonin; when there is light, the production of melatonin drops. Melatonin has strong antioxidant effects. Preliminary evidence suggests it may help strengthen the immune system. Melatonin-based food supplements have been proposed for a number of disturbances. Melatonin is widely available as tablets, capsules, cream and lozenges that dissolve under the tongue. Different people will have different responses to it. Lower doses appear to work better in people who are especially sensitive. Higher doses may cause anxiety and irritability. There exist several studies on the use of melatonin for the treatment of subjective tinnitus. In a US placebo controlled study on thirty patients having subjective tinnitus, melatonin has proven to be significantly more active in alleviating sleep disturbances caused by tinnitus compared to placebo. In another study on sixty-one subjects affected by chronic tinnitus, melatonin is associated with a statistically significant decrease in tinnitus intensity and improved sleep quality compared to placebo. In a recent review based on published basic science and clinical reports, tinnitus has been assessed in patients who had received melatonin using a visual analogue scale or the Tinnitus Handicap Inventory. Compared to a mixture of antioxidants which included tocopherol, ascorbate, glutathione and N-acetyl-cysteine, melatonin was estimated to be up to 150 times more effective in limiting cochlear side effects, evaluated using oto-acoustic emission distortion products, of gentamicin, tobramycin and cisplatin.

However, tinnitus in all of its forms, including subjective and chronic tinnitus, tinnitus induced by damage to cochlear nerve by chemical or physical agents, such as loud noise, does not have a satisfactory therapy hitherto for its amelioration or prevention, but it appears to benefit, to various degrees, from the use of the above mentioned and other substances, mostly of natural source, at varying concentrations and times. So, there still is a need for a satisfactory treatment of tinnitus.
It is the object of this invention to provide a unique composition to be administered for use as an oral medicament for treatment of hearing diseases, particularly tinnitus, achieving a satisfactory relief thereof, satisfying said need thanks to the synergy between specific substances.

As mentioned, the use has been only known hitherto of zinc, ALA and melatonin, singularly, as separate medicaments for relieving hearing disturbances, with poor results.

The inventors of the present application have found that a pharmaceutical formulation consisting of zinc, ALA and melatonin achieved surprisingly good results when administered to patients having a newly diagnosed tinnitus. Based on said finding, the present invention teaches a unique formulation that achieved the aforesaid object thanks to the synergism of alpha-lipoic acid, zinc, and melatonin.

Therefore, it is the subject-matter of the present invention a formulation for use as an oral medicament for the treatment of hearing diseases, including tinnitus, according to Claim 1. It includes, in combination: alpha-lipoic acid; zinc, and melatonin. It is particularly envisaged that in such a solid formulation, as set forth in Claim 2, said alpha-lipoic acid, zinc, and melatonin, are combined in the relative proportions of 100 to 1200 milligrams alpha-lipoic acid, 5.0 to 12.5 milligrams zinc and 0.5 to 3.0 milligrams melatonin.

It is further the subject-matter of the present invention an oral formulation including 100 to 1200 milligrams alpha-lipoic acid, 5.0 to 12.5 milligrams zinc and 0.5 to 3.0 milligrams melatonin for use as an oral medicament for the treatment of hearing disorders, including tinnitus.

The present invention will be fully understood based on the following disclosure.

Dietary reference intakes for zinc are: adolescents and adults: males, age 14 years and over: 11 mg/day, females, age 19 years and over: 8 mg/day. Zinc supplements in large amounts may cause diarrhea, abdominal cramps, and vomiting, usually within 3 - 10 hours of swallowing the supplements. The symptoms disappear within a short period of time after supplements discontinuation. Based on the above, the range of zinc in a formulation for tinnitus may range from 5 to 12.5 mg/day.

ALA, as food supplement alone, may be administered at doses in the range of 100 mg to 1200 mg/day.

Melatonin has been proposed for a wide range of dysfunctions. In the studies where melatonin was used for suppressing tinnitus, the dosage was invariably of 3 mg/day.

However, the above dosages are only indicative for ALA, zinc and melatonin used singly, not in combination.

The present invention reveals the following in a combined use of zinc; ALA, and melatonin, in both a solid and a liquid formulation.

Relative dosages of the three combined components of the formulation of the present invention may be as follows:

**TABLE I**

| **Ingredient** | **Daily dose (mg)** |
|---|---|
| ALA | 100 - 1,200 |
| Zinc | 5 - 12.5 |
| Melatonin | 0.5 - 3.0 |

Particularly, the present invention envisages an oral dose formulation, both solid and liquid, including ALA, zinc and melatonin in the dosages according to TABLE I.

Units of an initial batch of this invention have been used by an Italian ear, nose and throat specialist in a clinical study on patients affected by newly diagnosed tinnitus.

The aim of the study was to evaluate the efficacy and safety of a unique combination of ALA, zinc and melatonin administered orally for the treatment of patients with less than 3 months history of tinnitus and mono or bilateral hearing loss.

20 patients were initially submitted to hearing examination by impedance and audiometric controls, brainstem evoked potentials, evaluation of blood pressure, and tinnitus self-assessment tests (VAS 0-10 to increasing severity).

Patients were divided as follows: 11 patients with bilateral perceptive hearing loss of average degree for 4 and 8 kHz frequencies of the tonal scale with bilateral tinnitus (Group A), 5 patients with unilateral hearing loss of average entity for frequencies 4 and 8 kHz with ipsilateral tinnitus (Group B), 4 patients with mixed unilateral hearing loss of average entity for the mid frequencies 1 and 2 kHz and ipsilateral tinnitus (Group C).

Patients of all groups were subjected to sole therapy for tinnitus with tablets composed of the inventive association of alpha-lipoic acid, melatonin and zinc, administered orally, once daily for 60 days. At the end of treatment the tests performed at study start were repeated and results compared.

Statistically significant improvement of the perception of tinnitus and hearing thresholds compared to pre-treatment was declared and instrumentally demonstrated by the majority of patients of all of the 3 groups. In particular the reduction of the perception of tinnitus was always accompanied by an improvement of hearing thresholds. In group A (Total 11 patients), 5 patients reported a reduction in the disability caused by tinnitus but not its complete recovery. 2 patients reported complete recovery from tinnitus and hearing threshold normalization.

In group B (Total 5 patients), 1 patient reported a reduction of invalidity caused by tinnitus but not complete amelioration. 3 patients reported complete recovery from tinnitus and normalization of the hearing threshold.

In group C (Total 4 patients), 1 patient reported a reduction of invalidity caused by tinnitus but not complete amelioration. One patient reported complete recovery from tinnitus and normalization of hearing threshold.

The inventive three-component formulation under study allows for the possibility of simultaneous action on various aspects of the patho-physiology of the inner ear and tinnitus.

ALA is known for its antioxidant and neuroprotective properties, factors which are of primary importance in the case of acute injury such as tinnitus of recent onset and in the noble nervous tissues of the inner ear. It is also endowed of anti-inflammatory activity, as widely described in literature, of great advantage for the acute pathologies of the inner ear.

Melatonin is also of great scientific interest due to its many vasoactive and antioxidants properties. Its modulator activity on nervous tissues together with hemodynamic stabilization of the microcirculation of the inner ear could be the basis of its effectiveness on tinnitus. Its muscle relaxant action could explain the reduction in the transmission component in mixed hearing loss (group C) due to the relaxant action on the tensor muscle tone of the tympanum.

Zinc is present in many organs and tissues of the human body, including the cochlea where it reaches the highest concentration compared to all other parts of the body. A low level of serum zinc has been associated with the presence of tinnitus and implementation of this element with the formulation of the present invention may have a direct role in ameliorating this symptom.

It is clear that the synergistic action of the three components administered continuously for 2 months, has determined the positive results reported in comparison to results reported with each single component. Despite the limitations due to the small sample size, the results obtained by the inventors of the present application in this preliminary study, confirmed by hearing tests, are very encouraging, especially considering the known difficulties in the treatment of tinnitus and the significant discomfort it causes to patients.

The present invention has been disclosed with reference to examples thereof, but it is to be understood that variations can be made without departing from the scope of protection thereof.

Particularly, reference has been made in the clinical study to tablets, but the formulation of the present invention may be embodied in both solid and liquid formulations, particularly both solid and oral liquid dose formulations.

## Claims

1. Formulation of a food for special medical purposes or food supplement including active ingredients, wherein the active ingredients are:
alpha-lipoic acid, zinc, and melatonin, for use as an oral medicament for the treatment of hearing dysfunctions, including tinnitus.

2. Formulation of a food for special medical purposes or food supplement for use according to Claim 1, wherein said alpha-lipoic acid, zinc, and melatonin, are in the relative proportions of 100 to 1200 milligrams alpha-lipoic acid, 5.0 to 12.5 milligrams zinc and 0.5 to 3.0 milligrams melatonin.

3. Oral dose formulation of a food for special medical purposes or food supplement including 100 to 1200 milligrams alpha-lipoic acid, 5.0 to 12.5 milligrams zinc and 0.5 to 3.0 milligrams melatonin for use as an oral medicament for the treatment of hearing dysfunctions, including tinnitus.

4. Formulation of a food for special medical purposes or food supplement for use according to Claim 1, being a solid formulation.

5. Formulation of a food for special medical purposes or food supplement for use according to Claim 1, being a liquid formulation.

6. Oral dose formulation of a food for special medical purposes or food supplement for use according to Claim 3, being a solid formulation, including a tablet.

7. Oral dose formulation of a food for special medical purposes or food supplement for use according to Claim 3, being a liquid formulation.

## Patentansprüche

1. Formulierung eines Nahrungsmittels für besondere medizinische Zwecke oder Nahrungsergänzung, die aktive Wirkstoffe enthält, worin die aktiven Wirkstoffe send: alpha-Liponsäure, Zink und Melatonin, zur Verwendung als ein orales Medikament für die Behandlung von Dysfunktionen des Gehörs einschließlich Tinnitus.

2. Formulierung eines Nahrungsmittels für besondere medizinische Zwecke oder Nahrungsergänzung zur Verwendung
nach Anspruch 1, worin
die besagte alpha-Liponsäure, das besagte Zink und das besagte Melatonin in den relativen Anteilen von 100 bis 1200 Milligramm alpha-Liponsäure, 5,0 bis 12,5 Milligramm Zink und 0,5 bis 3,0 Milligramm Melatonin vorliegen.

3. Orale Dosierungsformulierung eines Nahrungsmittels für besondere medizinische Zwecke oder
Nahrungsergänzung umfassend 100 bis 1200 Milligramm alpha-Liponsäure, 5,0 bis 12,5 Milligramm Zink und 0,5 bis 3,0 Milligramm Melatonin zur Verwendung als ein orales Medikament für die Behandlung von Dysfunktionen des Gehörs einschließlich Tinnitus.

4. Formulierung eines Nahrungsmittels für besondere medizinische Zwecke oder Nahrungsergänzung zur Verwendung
nach Anspruch 1, die eine feste Formulierung ist.

5. Formulierung eines Nahrungsmittels für besondere medizinische Zwecke oder Nahrungsergänzung zur Verwendung
nach Anspruch 1, die eine
flüssige Formulierung ist.

6. Orale Dosierungsformulierung eines Nahrungsmittels für besondere medizinische Zwecke oder Nahrungsergänzung zur Verwendung
nach Anspruch 3,
die eine feste Formulierung ist, die eine Tablette einschließt.

7. Orale Dosierungsformulierung eines Nahrungsmittels für besondere medizinische Zwecke oder Nahrungsergänzung zur Verwendung nach Anspruch 3,
die eine flüssige Formulierung ist.

## Revendications

1. Formulation d'un aliment à des fins médicales spéciales ou d'un complément alimentaire comprenant des ingrédients actifs, dans laquelle les
ingrédients actifs sont :
acide alpha lipoïque, zinc, et mélatonine, pour une utilisation comme médicament par voie orale pour le traitement des troubles auditifs, notamment des acouphènes.

2. Formulation d'un aliment à des fins médicales spéciales ou d'un complément alimentaire pour une utilisation
selon la revendication 1, dans laquelle
lesdits acide alpha lipoïque, zinc, et mélatonine sont dans les proportions relatives 100 à 1 200 milligrammes d'acide alpha lipoïque, 5,0 à 12,5 milligrammes de zinc et 0,5 à 3,0 milligrammes de mélatonine.

3. Formulation d'une dose orale d'un aliment à des fins médicales spéciales ou d'un complément alimentaire comprenant 100 à 1 200 milligrammes d'acide alpha lipoïque, 5,0 à 12,5 milligrammes de zinc et 0,5 à 3,0 milligrammes de mélatonine pour une utilisation comme médicament par voie orale pour le traitement de troubles auditifs, notamment les acouphènes.

4. Formulation d'un aliment à des fins médicales spéciales ou d'un complément alimentaire pour une utilisation
selon la revendication 1, s'agissant d'une formulation solide.

5. Formulation d'un aliment à des fins médicales spéciales ou d'un complément alimentaire pour une utilisation
selon la revendication 1, s'agissant d'une formulation liquide.

6. Formulation d'une dose orale d'un aliment à des fins médicales spéciales ou d'un complément alimentaire pour une utilisation
selon la revendication 3,
s'agissant d'une formulation solide, comprenait un comprimé.

7. Formulation d'une dose orale d'un aliment à des fins médicales spéciales ou d'un complément alimentaire pour une utilisation selon la revendication 3,
s'agissant d'une formulation liquide.
